(19) Europäisches Patentamt

European Patent Office

Office européen des brevets

(11) Publication number: **0 177 456**
**B1**

(12) # EUROPEAN PATENT SPECIFICATION

(45) Date of publication of patent specification: **02.08.89**

(21) Application number: **85830029.6**

(22) Date of filing: **13.02.85**

(51) Int. Cl.⁴: **A 61 M 7/00**

(54) Bottle provided with cap and warranty seal particularly designed for vaginal washings and enemas.

(30) Priority: **05.10.84 IT 2303884**

(43) Date of publication of application:
**09.04.86 Bulletin 86/15**

(45) Publication of the grant of the patent:
**02.08.89 Bulletin 89/31**

(84) Designated Contracting States:
**AT BE CH DE FR GB LI NL SE**

(56) References cited:
**FR-A-2 123 309**
**US-A-4 200 097**

(73) Proprietor: **PLASTIAPE S.a.s. di Magni E.,**
**Citterio G. & C.**
**Via Stoppani, 1/3**
**I-22058 Osnago (Como) (IT)**

(72) Inventor: **Citterio, Gianfranco**
**Via Stoppani, 1/3**
**I-22058 Osnago(Como) (IT)**

(74) Representative: **Cicogna, Franco**
**Ufficio Internazionale Brevetti Dott.Prof. Franco**
**Cicogna Via Visconti di Modrone, 14/A**
**I-20122 Milano (IT)**

Courier Press, Leamington Spa, England.

## Description

The present invention relates to a bottle provided with a warranty seal and with a cap, specifically designed for vaginal washings and enemas.

As it is well known, ready to use solutions are presently available for making vaginal washings or enemas; those solutions are presently held in vessels which are generally provided with a flexible cannula extending from the vessel or bottle closure plug and provided, at the top, with a removable portion for tightly closing said cannula.

In those embodiments, it is hardly possible to preserve the required hygienic conditions, since the user has to necessarily manipulate the cannula, with pollution risks.

Another drawback of the prior art approaches is that the mentioned bottles present packaging problems, because of the outwardly extending cannula size.

Other known approaches are of complex structure and not effective to meet the user needs.

For example the US—A—4 200 097 Patent discloses a bottle for vaginal washing or enemas comprising a vessel containing a washing solution, the mouth of the vessel being closed by a plug member held in position by a ring member, a withdrawable elongate cannula mounted in the plug member and extending outwardly of the vessel, the cannula being slidable between a first retracted position and a second extended position, a supply head at the free end of the cannula, and valve means for controlling flow of the washing solution through the cannula. In this known bottle, however, the cannula is not perfectly protected against contamination.

Accordingly, the task of the present invention is to obviate the above mentioned drawbacks, by providing such a bottle, provided with cap and warranty seal, which is specifically designed for vaginal washings and enemas and effective to preserve great hygienic conditions.

Within that task, it is an object of the present invention to provide such a bottle which may be used without the need of manipulating the associated cannula.

Another object of the present invention is to provide such a bottle which is of easily packaging, thereby simplifying all of the related operations.

Yet another object of the present invention is to provide such a bottle including means for preventing pollutions to the solution held therein.

Yet another object of the present invention is to provide such a bottle which is simple to be made and which, moreover, is of reduced cost.

According to one aspect of the present invention, the above task and objects, as well as yet other objects which will become more apparent thereinafter are achieved by a bottle for vaginal washing and enemas having the characteristics of the characterising portion of claim 1.

To the mentioned plug member there is slidingly coupled a withdrawable cannula provided, on the outside of said vessel, with a metering or supplying head, including closure and check valve means.

Further characteristics and advantages of the present invention will become more apparent from the following detailed description of a preferred embodiment thereof, given by way of example, being illustrated in the accompanying drawings, where:

Figure 1 illustrates a possible embodiment of the vessel;

Figure 2 illustrates the ring member with the cap provided with the warranty seal;

Figure 3 illustrates, by a partial cross-section view, the plug member with applied the cap;

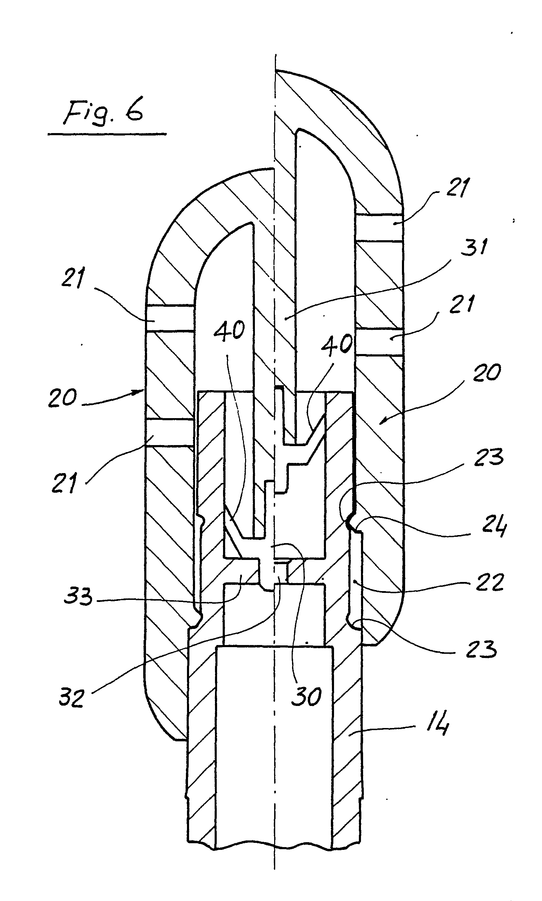

Figure 4 is a top plan view illustrating the associated cap and ring member;

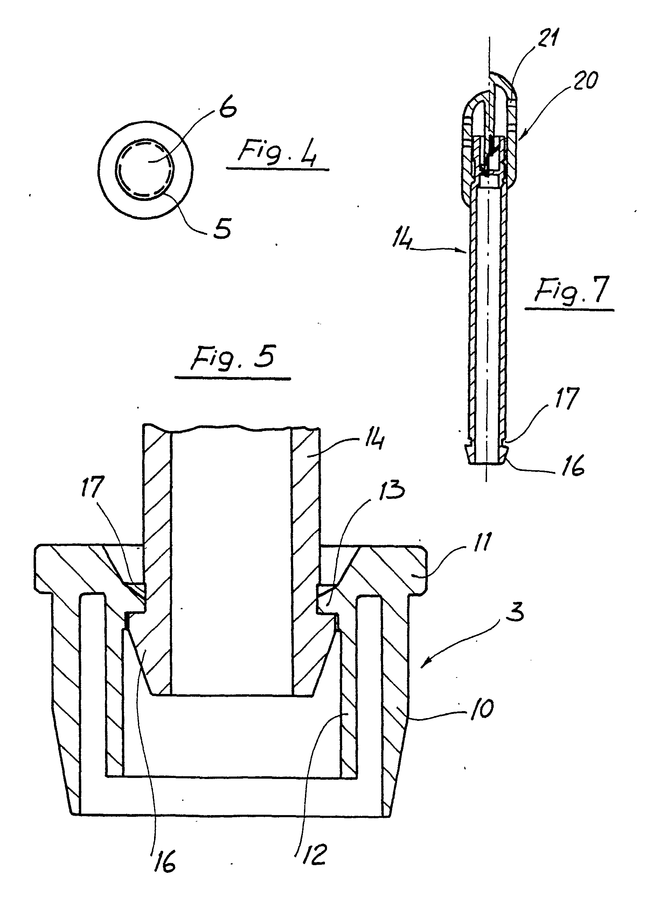

Figure 5 is a cross-section view illustrating the detail of the plug member and of an abutment for preventing the cannula from being withdrawn;

Figure 6 is a cross-section view illustrating two different operative positions of the supplying or metering head as arranged at the end of the cannula; and

Figure 7 illustrates a cross-sectional view of the cannula assembly.

With reference to the above mentioned Figures, the bottle provided with cap and warranty or antitampering seal according to the present invention comprises a vessel, indicated overally at the reference number 1 which is provided, at the top, with a mouth provided with annular outer ridges 2.

At the bottle mouth there is pressure fitted a plug member, indicated overally at the reference number 3, which is held in position by a ring member 4 coupled to the mentioned ridges 2.

On the top of said ring member 4 there is coupled, through tearable portions 5, a cap 6 made of a flexible material and having an elongated shape.

The plug member is provided with an outer skirt 10, effective to be pressure inserted inside the mouth of the vessel 1 and coupled at the top to an annular flange 11 abutting against said mouth and being held in position by the ring member 4.

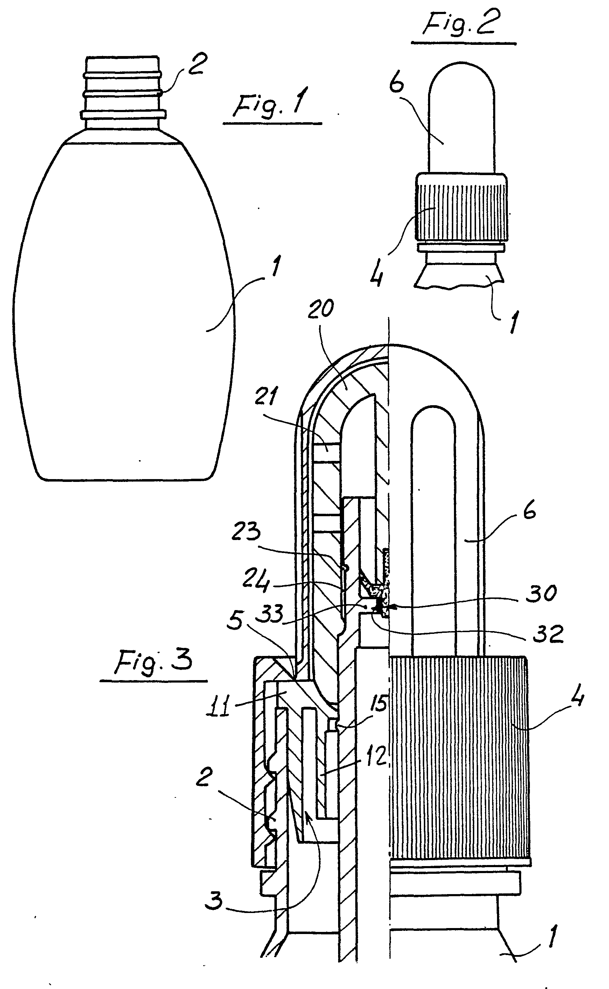

The plug member 3 is moreover provided with an inner skirt 12 comprising at the top an annular port or opening defined by an abutment ring 13, inside of which there is slidingly housed a withdrawable cannula 14, of elongated shape, which is provided, at its top end, as it should be apparent from Figure 3, with a stop dog 15 and, at the bottom end whereof, with an abutment portion 16 arranged under an annular groove 17.

Said stop dog 15 is effective to hold said cannula in the inside of the vessel, as the latter is sealed, and affords the possibility of withdrawing it as the cannula projecting from the plug member is withdrawn, to engage the abutment portion with the abutment ring 13 which, in turn, is inserted in said annular groove 17, thereby providing a tight closure between the outer surface of the cannula and the plug member.

At the top end of the cannula 14 there is provided a metering or supplying head, indicated overally at 20, which is provided with holes 21 and is preferably of elongated and rounded shape.

The supplying head 20 is coupled to the top end of the cannula, with the possibility of assuming different positions as defined by the translation of said head with respect to the axial extension of said cannula.

In order to obtain said displacement, at the top of the cannula 14 there is provided, on its outer surface, an annular recess 22 provided, at the ends thereof, with recessed portions 23 thereinto an annular lug 24 is inserted as formed on the inner surface of the supplying head 20 which, depending on the recess 23 being engaged with the annular lug 24, assumes either an opening or a closure position.

At the top end of the cannula there are provided closure and check valve means which consists of a shutter element 30 coupled to an axial stem 31, provided inside said supply head 20 and effective to engage, at a first position, with a port 32 as defined on a diaphragm 33 arranged for closing said cannula.

With the shutter 30 inserted into the opening 32, the cannula is tightly closed.

By displacing the supply head 20 with respect to the cannula, the shutter 30 is moved away from the port 32, with a consequent opening of the communication between the outside and inside of the vessel 1.

With the shutter 30 there is associated a mushroom shaped body 40 which extends through the overall circumference of the cannula and practically provided a check valve, since it is slanted in such a way to allow for the liquid inside the vessel to exit therefrom, whereas, by adhering to the inner walls of the cannula, it prevents the fluid from flowing in the opposite direction.

The operation of the bottle according to the present invention is a very simple one.

In fact, in order to open said bottle, it is sufficient to tear the cap 6, in such a way as to break the portions 5.

Then, by exploiting the flexibility of said cap, it will be possible, by a slight pressure, to clamp said cap practically against the supply head 20 and provide a withdrawal action to cause the cannula to be removed as far as the abutment portion 16, as provided at the bottom end of the cannula, engages with the abutment ring 13 of the plug member.

Successively, by a further pulling action, the supply head will be displaced and brought from the closure position to the solution metering position.

Upon having carried out the mentioned simple operations, which are obtained by simply acting on the outer surface of the cap, which will be then disposed of, the cannula may be readied for operation, without the need of manipulating portions of said cannula.

Moreover warranty of antitampering seals are provided effective to assure the proper preservation of the washing solution as well as valve means for perfectly sealing said washing solution.

Another main feature of the present invention consists of the fact that said valve means are so designed as to also provide a check valve which prevents the used solution or liquid from flowing back to the inside of the bottle, thereby preventing any pollution.

The invention as disclosed is susceptible to several modifications and variations all whereof come within the scope of the invention as defined by the claims.

In practicing the invention the used materials, as well as the contingent shape and size, may be any according to requirements.

**Claims**

1. A bottle for vaginal washing or enemas comprising a vessel (1) containing a washing solution, the mouth of the vessel being closed by a plug member (3) held in position by a ring member (4), a withdrawable elongate cannula (14) mounted in the plug member and extending outwardly of the vessel, the cannula being slidable between a first, retracted position and a second, extended position, a supply head at the free end of the cannula, and valve means (30) for controlling flow of the washing solution through the cannula, characterized in that the bottle further comprises a flexible protective cap (6) coupled to the ring member by tearable means (5) and enclosing the cannula in the retracted position thereof, thus permitting the cannula to be manually withdrawn to its extended position whilst avoiding manual contact with the cannula, and the valve means acts as a check valve means for preventing liquid from flowing back into the vessel in use.

2. A bottle according to Claim 1, characterized in that said plug member is provided with an outer skirt (10) which is pressure inserted into said vessel and with an inner skirt (12) defining the passage opening for said cannula, an abutment ring (13) being provided at said opening.

3. A bottle according to either of the preceding claims, characterized in that said cannula, at its free end, is provided with a flange (15) effective to engage with the abutment ring member in such a way as to hold said cannula in said vessel when the latter is in its closed condition, and in that said cannula, at its inner end, is provided with an abutment portion (16) arranged under an annular groove (17) effective to engage with said abutment ring in such a way as to prevent said cannula from being further withdrawn from said plug member, said annular groove being effective to be tightly engaged by the abutment ring.

4. A bottle according to one or more of the preceding claims, characterized in that said supply head (20) is coupled to said cannula in such a way as to be axially slidable from a first closure position to a second washing solution supplying position.

5. A bottle according to Claim 4, characterized in that said cannula is provided, near its free end, with an annular recess (22) provided, at its axial ends, with recessed annular portions (23) engageable with a lug (24) as defined by the inner surface of the supply head, for locating the latter to the closure position or to the supply position.

6. A bottle according to one or more of the preceding claims, characterized in that said closure and check valve means consist of a shutter member (30), coupled to an axially extending stem (31), which extends inside said supply head and is engageable with a port (32), extending through a closure diaphragm (33), arranged at the free end of said cannula, from said shutter member there extends a mushroom-shaped member (40) having slanted lips engaging with the inner surface of said cannula in order to allow for the washing solution to flow from the vessel to the outside but not in the opposite direction.

## Patentansprüche

1. Spülfläche für Scheidenspülung oder Klistier, mit einem Gefäss (1) für die Lösung, wobei die Mündung des Gefäss mit einem von einem Stellring (4) gehaltenen Stopfen (3) geschlossen ist, einer herausziehbaren verlängerten in dem Stopfen eingelegten und aussen dem Gefäss sich erstreckenden Kanüle, wobei die Kanüle zwischen einer ersten Zurückgezogenen Stellung und einer zweiten herausgezogenen Stellung verschiebbar vorgesehen ist, einem auf der freien Seiten der Kanüle angeordneten Versorgungsteil und Ventilmitteln (30) für die Regelung des Lösungsfluss durch die Kanüle, dadurch gekennzeichnet, dass ausserdem die Fläche eine biegsame Verschlusskappe einschliesst, die durch Reissmitteln (5) mit dem Stellring gekoppelt ist und die die Kanüle in seiner zurückgezogenen Stellung einfässt, wobei die Handherausziehung der Kanüle begünstigt und gleichzeitig die Berührung derselben Kanüle von Hand verhindert wird, und das Ventilmittel, um so die Zurückfliessen der Lösung in der benutzten Flasche zu verhindern, als Rückschlagventil wirkt.

2. Spülflasche nach Anspruch 1, dadurch gekennzeichnet, dass das obengenannte Stopfenglied mit einer in dem obengenannten Gefäss eingepressten Aussenkleidung (10) vorgesehen ist, wobei die Innenkante (12) desselben Stopfenteil eine Durchgangsöffnung für die Kanüle bestimmt, und an deren Öffnung einen Abdichtungsring vorgesehen ist.

3. Flasche nach einer der vorhergehenden Ansprüchen, dadurch gekennzeichnet, dass die obengenannte Kanüle an seiner freien Ende mit einem Flansch (15) vorgesehen ist, wobei dieser Flansch, um so die obengenannte Kanüle als das obengenannte Gefäss in seiner geschlossenen Stellung steht dasselbe Gefäss zu halten, mit dem Abdichtungsring eingreift, dadurch gekennzeichnet, dass diese Kanüle an seiner Innenende mit einer unter einer mit dem obengenannten Abdichtungsring eingreifbaren Rille (17) angeordneten Halteporzion (16) um so zu verhindern, dass die obengenannte Kanüle von dem obengenannten Stopfen weiterherausgezogen wird, wobei die obengenannte Ringrille von dem Abdichtungsring festeingreifbar ist.

4. Flasche nach einem oder mehreren vorhergehenden Ansprüchen, dadurch gekennzeichnet, dass die Versorgungskopf (20) von einer ersten geschlossenen Stellung an einer zweiten Versorgungsstellung der Spüllösung mit obengenannten Kanüle axial verschiebbar gekoppelt ist.

5. Flasche nach Anspruch 4, dadurch gekennzeichnet, dass die obengenannte Kanüle an seiner freien Ende mit einer Ringrille (22) vorgesehen ist, die an seiner axialen Ende mit Ringrillenportionen (23) versehen ist, wobei diese mit einer von der Innenfläche des Versorgungskopf bestimmten Rippe (24) eingreifen, um so dieses Versorgungskopf in seiner geschlossenen Stellung oder Versorgungsstellung zu anordnen.

6. Flasche nach einer oder mehreren vorhergehenden Ansprüchen, dadurch gekennzeichnet, dass die obengenannte Verschlusskappe oder Ventil aus einer mit einem axial sich erstreckenden Spindel gekoppelten Deckelglied (30), das innenwärts dem obengenannten Versorgungskopf erstreckt sich, und mit einer durch eine Schlussblende (30) sich erstreckenden Öffnung (32) bestehen, wobei die Schlussblende an der freien Ende der Kanüle angeordnet ist, und wobei von dem obengenannten Deckelglied ein pilzförmiges Glied (10) erstreckt sich, deren geneigten Kanten mit den Innenfläche der obengenannten Kanüle eingreifen, um so das Aussenwärtsdurchluss der Spüllösung aus dem Gefäss zu erleichtern, aber nicht gegenwärts.

## Revendications

1. Irrigateur pour douche vaginale ou clystère, comprenant un récipient (1) pour la solution, l'embouchure du récipient étant fermée par un bouchon (3) tenu en position par une bague (4), une canule allongée à extraire (14) montée dans le bouchon et s'étendant à l'extérieur du récipient, la canule étant coulissante entre une première position retirée et une deuxième position étendue, une partie d'alimentation sur le côté libre de la canule et moyens (30) à soupape pour régler l'écoulement de la solution à travers la canule, caractérisé en ce que l'irrigateur comprends en outre un capouchon (6) de protection flexible accouplé à l'anneau par de moyens à accroc (5) et embrassant la canule dans sa position retirée, en permettant ainsi l'extraction à la main de la canule et en même temps en empêchant le contact à la main de la canule, et moyens à soupape agissent comme moyens à soupape de contrôle pour éviter que le liquide retourne dans le récipient en usage.

2. Irrigateur selon la revendication 2, caractérisé en ce que ledit moyen à accroc est pourvu d'un revêtement extérieur (10) introduit à pression dans ledit récipient et avec un bord intérieur (12) définant l'embouchure de passage pour ladite canule, une bague d'arrêt (13) étant prévue à ladite embouchure.

3. Irrigateur selon chacune des revendications précédentes, caractérisé en ce que ladite canule dans son extrémité libre est munie d'une bride (15) en état de s'engager avec la bague d'arrêt ainsi de retenir ladite canule dans ledit récipient comme ceci se trouve dans sa position fermée, et en ce que ladite canule dans son extrémité intérieure est munie d'une portion (16) d'arrêt ainsi d'empêcher que ladite canule soit extraite encore dudit bouchon, ladite rainure annulaire (17) se pouvant engager fort par la bague d'arrêt sous laquelle elle est située.

4. Irrigateur selon une ou plusieurs des revendications précédentes, caractérisé en ce que ladite tête d'alimentation (20) est accouplée à ladite canule de façon de pouvoir coulisser axialement d'une première position fermée à une deuxième position d'alimentation de la solution d'irrigation.

5. Irrigateur selon la revendication 4, caractérisé en ce que ladite canule est pourvue, près de son extrémité libre, d'une rainure annulaire (22) qui est pourvue, dans ses extrémités axiales, de portions annulaires creusées (23) qui peuvent s'engager par une ailette (24) comme définie par la surface intérieure de la tête d'alimentation, pour placer cette-ci dans sa position de fermeture ou dans sa position d'alimentation.

6. Irrigateur selon une ou plusieurs des revendications précédentes, caractérisé en ce que lesdits moyens de fermeture et à soupape de contrôle se forment d'un moyen à porte (30) accouplé à une baguette (31) s'étendant axialement, qui s'étend à l'intérieur de ladite tête d'alimentation et peut s'engager avec une embouchure (32) s'étendant à travers un diaphragme (33) de fermeture, situé à l'extrémité libre de ladite canule, dudit moyen à porte s'étend un moyen fongiforme (40) ayant bords inclinés s'engageant avec la surface intérieure de ladite canule dans le but de permettre que la solution d'irrigation coule du récipient à l'extérieur, mais non pas au contraire.

EP 0 177 456 B1

Fig. 2

Fig. 1

Fig. 3

Fig. 6

EP 0 177 456 B1

Fig. 4

Fig. 5

Fig. 7

3